(19) Europäisches Patentamt
European Patent Office
Office européen des brevets

(11) EP 1 344 062 B1

(12) **FASCICULE DE BREVET EUROPEEN**

(45) Date de publication et mention de la délivrance du brevet:
**08.04.2009 Bulletin 2009/15**

(21) Numéro de dépôt: **01994906.4**

(22) Date de dépôt: **19.12.2001**

(51) Int Cl.:
***G01N 33/543*** *(2006.01)* ***C12Q 1/68*** *(2006.01)*
***G01N 21/64*** *(2006.01)*

(86) Numéro de dépôt international:
**PCT/FR2001/004068**

(87) Numéro de publication internationale:
**WO 2002/050540 (27.06.2002 Gazette 2002/26)**

(54) **SUPPORT POUR DETERMINER UN ANALYTE COMPORTANT UN FILTRE SPECTRAL SELECTIF**

TRÄGER MIT EINEM SELEKTIVEN SPEKTRALFILTER ZUR BESTIMMUNG EINES ANALYTEN

SUPPORT FOR DETERMINING AN ANALYTE COMPRISING A SELECTIVE SPECTRAL FILTER

(84) Etats contractants désignés:
**AT BE CH CY DE DK ES FI FR GB GR IE IT LI LU MC NL PT SE TR**

(30) Priorité: **20.12.2000 FR 0016650**

(43) Date de publication de la demande:
**17.09.2003 Bulletin 2003/38**

(73) Titulaire: **COMMISSARIAT A L'ENERGIE ATOMIQUE**
**75015 Paris (FR)**

(72) Inventeurs:
- **CHATON, Patrick**
**F-38570 THEYS (FR)**
- **VINET, Françoise**
**F-38000 GRENOBLE (FR)**

(74) Mandataire: **Poulin, Gérard et al**
**Brevalex**
**3, rue du Docteur Lancereaux**
**75008 Paris (FR)**

(56) Documents cités:
**WO-A-91/04491** **WO-A-92/16826**
**FR-A- 2 801 977** **US-A- 5 639 671**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 344 062 B1

## Description

### Domaine technique

La présente invention a pour objet un support d'analyse ou "biopuce" destiné à la détermination d'analytes tels que des cibles ADN ou ARN, des protéines, des anti-gènes et des anti-corps.

De façon plus précise, elle concerne des biopuces dont le principe repose sur la détection d'une réaction spécifique de liaison de l'analyte avec un ligand spécifique de cet analyte et de détection de cette réaction au moyen de marqueurs fluorescents.

Ces biopuces trouvent des applications dans de nombreux domaines, en particulier en biologie pour le séquençage des génomes, la recherche des mutations, le développement de nouveaux médicaments, etc.

### Etat de la technique antérieure

Dans le cas où l'analyte est une cible biologique de type ADN ou ARN, un support d'analyse de ce type comprend une pluralité de sondes oligonucléotidiques susceptibles de donner lieu à une hybridation avec les cibles biologiques à analyser. L'hybridation correspond à l'appariement des brins-cibles avec les brins d'ADN complémentaires disposés sur le support. Pour déterminer la nature des cibles, il est donc nécessaire de pouvoir situer quels sont les sites du support et donc les oligonucléotides qui ont donné lieu à une hybridation.

Le document Médecine/Sciences, vol. 13, n°11, 1997, pp. 1317-1324 [1] décrit des supports d'analyse de ce type.

Habituellement, on détermine l'hybridation des cibles biologiques sur le support à l'aide d'un marqueur fluorescent qui est associé aux cibles biologiques, après extraction des zones intéressantes (lyse et amplification éventuelle).

Après mise en contact des cibles marquées avec le support d'analyse comportant les oligosondes, on détermine les sites où a lieu l'hybridation en réalisant une excitation de l'ensemble des marqueurs fluorescents, suivie d'une lecture des sites pour détecter la lumière de fluorescence réémise par les marqueurs. Les sites pour lesquels une lumière de fluorescence est détectée sont ceux qui ont fixé des molécules-cibles.

La détection est assurée par des microscopes ou des scanners confocaux de type "General Scanning" ou "Genetic Microsystem". Des systèmes de lecture adaptés à diverses biopuces sont décrits par exemple dans US-A-5,578,832 [2] et US-A-5, 646, 411 [3].

Ce principe de détection des cibles par marquage fluorescent est robuste et simple à mettre en oeuvre ; par ailleurs, il présente une sensibilité de détection ultime car des cibles uniques peuvent être mises en évidence sans une instrumentation lourde.

Généralement, on utilise pour cette détection un seul marqueur fluorescent qui est associé aux cibles biologiques. Toutefois, il serait intéressant d'utiliser simultanément plusieurs marqueurs fluorescents car cette redondance permet de détecter de manière non ambiguë la cible biologique, ce qui est particulièrement intéressant pour étudier l'expression des gènes et confirmer la signature du gène étudié.

Toutefois, lorsque l'on utilise deux marqueurs fluorescents, l'un des marqueurs peut avoir un rendement de luminescence plus important, et par là même, il peut être incompatible avec un second marqueur fluorescent d'intensité plus faible. Une correction du niveau de fluorescence du marqueur le plus intense est difficile à mettre en oeuvre car elle agit directement sur la densité des cibles et est de ce fait difficilement maîtrisable.

Par ailleurs, les scanners de lecture présentent une sélectivité spectrale entre les fluorophores, ce qui peut fausser l'analyse des signaux de la biopuce. La modification des scanners est difficile pour le non spécialiste car ce sont la plupart du temps des appareils commerciaux et leur adaptation aux marqueurs fluorescents choisis est une démarche qui peut s'avérer très lourde.

### Exposé de l'invention

La présente invention a précisément pour objet un support d'analyse conçu de façon à compenser la différence de luminescence entre plusieurs marqueurs fluorescents, qui peut de plus s'adapter aux réponses spectrales des scanners de lecture.

Aussi, l'invention a pour objet un support d'analyse pour déterminer un analyte par mise en oeuvre d'une réaction spécifique de liaison dudit analyte avec un ligand spécifique de cet analyte, et détermination de la réaction de liaison au moyen d'au moins deux marqueurs fluorescents présents sur l'analyte, cette détermination étant effectuée en appliquant un champ excitateur et en détectant l'émission fluorescente des différents marqueurs fluorescents, ledit support comprenant un substrat sur lequel sont fixés une pluralité de ligands spécifiques de l'analyte à déterminer, ledit substrat étant revêtu d'une ou plusieurs couche(s) de matériau(x) formant un ensemble capable d'atténuer ou d'augmenter le champ excitateur pour au moins l'un des marqueurs fluorescents par rapport aux autres, et les ligands étant

fixés sur la dernière couche de l'ensemble.

On précise que l'on entend par "analyte", tout composé chimique ou biochimique capable de présenter une réaction de liaison avec un ligand spécifique de cet analyte. A titre d'exemple d'analyte, on peut citer les cibles biologiques telles que les oligonucléotides, soit les sondes ADN ou ARN, les protéines et leurs récepteurs, et les composés immunologiques du type antigène et anticorps.

Les couples analyte-ligand spécifique de l'analyte peuvent être choisis parmi les couples suivants : oligonucléotide-oligonucléotide complémentaire, anticorps-antigène, protéine-récepteur de protéine, ou l'inverse.

Selon l'invention, le substrat du support d'analyse est revêtu d'une ou plusieurs couches réalisées en un ou plusieurs matériaux formant un ensemble capable d'atténuer ou d'augmenter le champ excitateur pour au moins l'un des marqueurs fluorescents. La ou les couches(s) forment un ensemble jouant le rôle de filtre optique qui présente des caractéristiques de filtrage optique, tout en étant transparent aux longueurs d'ondes d'excitation et d'émission des marqueurs fluorescents.

Il convient donc de choisir la nature du (des) matériau(x) constituant la ou lesdites couches, ainsi que l'épaisseur de la ou des couches pour obtenir cette atténuation ou cette augmentation du champ excitateur pour au moins l'un des marqueurs fluorescents par rapport aux autres.

L'épaisseur de chaque couche est choisie en fonction de l'indice de réfraction n du matériau la constituant, à la longueur d'onde $\lambda$ du maximum d'absorption du marqueur fluorescent.

Ainsi, dans le cas où on utilise une seule couche mince, l'épaisseur e de cette couche doit satisfaire la règle suivante :

$$en_1 = k\lambda_{M1}/4$$

où $n_1$ est l'indice de réfraction du matériau à la longueur d'onde $\lambda_{M1}$ du maximum d'absorption du marqueur fluorescent $M_1$, et k est un entier impair, pour augmenter l'excitation du marqueur fluorescent M1.

Dans le cas où on utilise plusieurs couches minces, le calcul des épaisseurs souhaitées pour ces couches minces peut être effectué en suivant l'enseignement du document Edward H. Hellen et Daniel Axelrod, J. Opt. Soc. Am. B, vol. 4, n°3, March 1987, p. 337 à 350 [4].

Les épaisseurs sont de l'ordre de grandeur de la longueur d'onde d'excitation ou d'émission des marqueurs fluorescents utilisés.

La ou les couches minces ont une haute qualité optique et elles présentent des caractéristiques de filtrage optique.

Les matériaux des couches minces peuvent être choisis parmi les oxydes réfractaires, les fluorures métalliques et les oxynitrures de silicium.

A titre d'exemple d'oxyde réfractaire, on peut citer $TiO_2$, $HfO_2$, $Ta_2O_5$, $SiO_2$ et $ZrO_2$.

A titre d'exemple de fluorure, on peut citer $YF_3$ et $MgF_2$.

Le matériau de la dernière couche sur laquelle seront fixés les ligands spécifiques de l'analyte à déterminer est choisi de façon à permettre cette fixation.

Le matériau de la première couche qui est directement en contact avec le substrat, est choisi de façon à être compatible avec le substrat.

Les substrats utilisés peuvent être de type divers. Généralement, on les réalise en semi-conducteur, en verre ou en matière plastique.

Dans le cas où l'on utilise un substrat de silicium, la couche d'atténuation ou d'augmentation du champ excitateur est avantageusement réalisée en silice $SiO_2$.

Bien entendu, le ou les matériaux formant la ou les couches sont choisis en fonction des marqueurs fluorescents utilisés de façon à permettre cette atténuation ou cette augmentation et à assurer l'équilibrage des signaux émis par ces marqueurs.

Selon un mode préféré de réalisation de l'invention, le support est adapté à la détermination des signaux émis par deux marqueurs fluorescents.

On peut utiliser dans ce but différents types de marqueurs, par exemple le premier marqueur fluorescent peut être Cy3 dont la longueur d'onde d'excitation est d'environ 550 nm et la longueur d'onde d'émission est d'environ 580 nm, et le second marqueur fluorescent peut être Cy5 dont la longueur d'onde d'excitation est d'environ 650 nm et la longueur d'onde d'émission est d'environ 680 nm.

Avec ces marqueurs, on peut utiliser une couche de silice thermique ayant une épaisseur d'environ 100 à 1000 nm sur un substrat en silicium.

On peut déterminer les signaux émis par les marqueurs soit simultanément, soit successivement.

La présente invention a encore pour objet un procédé de fabrication d'un support d'analyse présentant les

caractéristiques données ci-dessus.

Ce procédé consiste :

1) à former sur un substrat une ou plusieurs couches minces de matériau(x) destinée(s) à former l'ensemble capable d'atténuer ou d'augmenter le champ d'excitation pour au moins l'un des marqueurs fluorescents, et
2) à fixer sur la dernière couche formée sur le substrat les ligands nécessaires à la détermination de l'analyte.

Généralement, la ou les couches de matériau(x) peuvent être formées sur le substrat par les techniques classiques de fabrication de couches minces optiques telles que le dépôt sol-gel, le dépôt chimique en phase vapeur (CVD), le dépôt physique en phase vapeur (PVD), le dépôt au moyen d'un canon à électrons, la pulvérisation cathodique, ou encore l'oxydation thermique du substrat.

Lorsque les ligands nécessaires à la détermination sont des oligonucléotides, ceux-ci peuvent être fixés sur la dernière couche formée sur le support par les techniques utilisées habituellement pour la réalisation de biopuces.

Ces techniques peuvent faire appel à des dispenseurs piézoélectriques qui déposent sur le substrat des gouttes contenant l'oligonucléotide, de quelques centaines de micromètres de diamètre, aux endroits voulus. On peut aussi utiliser des techniques basées sur l'emploi de polymères conducteurs qui immobilisent les sondes par copolymérisation comme il est décrit dans WO 94/22889 [5], ou des techniques de synthèse in situ de l'oligonucléotide selon lesquelles on fait croître les sondes base après base sur la dernière couche formée sur le substrat.

La synthèse in situ met en jeu les réactions classiques de couplage par l'intermédiaire des phosphoramidites, des phosphites ou des phosphonates, pour la condensation successive des nucléotides judicieusement protégés. Le cycle de synthèse comprend les étapes de déprotection, couplage, blocage et oxydation, et permet de faire croître l'oligonucléotide à partir de la surface de chaque site.

De préférence, on réalise un traitement préliminaire de la dernière couche pour assurer la fixation des ligands par l'intermédiaire de bras espaceurs et les éloigner ainsi de la surface du support. Ce traitement peut consister en un greffage d'un bras espaceur sur cette dernière couche.

Dans le cas où la dernière couche est en silice $SiO_2$, ce traitement peut consister en une fonctionnalisation par des groupements époxy, suivie d'une réaction avec un glycol.

D'autres caractéristiques et avantages de l'invention apparaîtront mieux à la lecture de la description qui suit d'exemples de réalisation donnés bien entendu à titre illustratif et non limitatif, en référence aux dessins annexés.

**Brève description des dessins**

- la figure 1 est un diagramme illustrant les spectres d'absorption de deux marqueurs fluorescents utilisés dans l'invention ;
- la figure 2 est un diagramme illustrant l'évolution des champs excitateurs en fonction de l'épaisseur (en nm) de la couche déposée sur le substrat pour les marqueurs fluorescents Cy3 et Cy5 ;
- la figure 3 est un diagramme illustrant l'évolution de l'intensité de fluorescence des marqueurs fluorescents Cy3 et Cy5 en fonction de l'épaisseur (en nanomètre) de la couche d'oxyde $SiO_2$ présente sur le substrat ; et
- la figure 4 représente l'évolution des propriétés de réflexion (en %) d'une structure multicouche conforme à l'invention en fonction de la longueur d'onde.

**Exposé détaillé des modes de réalisation**

Sur la figure 1, la courbe 1 représente le spectre d'absorption du premier marqueur fluorescent Cy3 utilisé dans l'invention, et la courbe 2 représente le spectre d'absorption du second marqueur fluorescent Cy5 utilisé dans l'invention, soit les rendements quantiques en fonction de la longueur d'onde d'excitation.

Sur cette figure, on voit que les spectres des deux marqueurs n'ont pas la même amplitude. Aussi, pour obtenir une réponse intéressante avec ces deux marqueurs, il convient, soit d'atténuer le champ d'excitation du second marqueur, soit d'augmenter le champ d'excitation du premier marqueur pour équilibrer les deux signaux émis par les marqueurs et obtenir une réponse satisfaisante.

**Exemple 1**

Dans cet exemple, on utilise une biopuce qui comprend un substrat en silicium sur lequel on forme une seule couche de silice thermique $SiO_2$. Un calcul électromagnétique effectué d'une façon similaire au calcul décrit dans le document [4], permet de déterminer les épaisseurs optimales d'excitation pour les deux marqueurs fluorescents utilisés,

Cy3 et Cy5 qui ont les caractéristiques suivantes :

Cy3 : longueur d'onde maximale d'absorption égale à 550 nm et longueur d'onde d'émission égale environ à 580 nm ;
Cy5 : longueur d'onde maximale d'absorption égale à 650 nm et longueur d'onde d'émission égale environ à 680 nm.

La figure 2 représente l'évolution calculée des champs excitateurs en fonction de l'épaisseur (en nanomètre) de la couche de silice thermique $SiO_2$, lorsqu'on réalise l'excitation aux longueurs d'ondes maximales d'absorption des deux marqueurs. Sur cette figure, on remarque qu'il existe des épaisseurs de silice qui permettent d'atténuer la fluorescence de Cy3 par rapport à celle de Cy5 et inversement.
A partir de ces calculs, on réalise plusieurs supports d'analyse avec des épaisseurs différentes de silice thermique.
Dans ce but, on utilise 10 substrats en silicium sur lesquels on forme une couche de silice par oxydation thermique afin d'obtenir des substrats ayant les épaisseurs suivantes de $SiO_2$ :

- 2 substrats avec une couche de 100 nm,
- 2 substrats avec une couche de 190 nm,
- 2 substrats avec une couche de 475 nm,
- 2 substrats avec une couche de 500 nm, et
- 2 substrats avec une couche de 545 nm.

On soumet ensuite les substrats à un nettoyage par immersion dans une solution alcaline de soude et on les rince dans de l'eau déionisée.
On fixe ensuite sur la couche de $SiO_2$ un bras espaceur en effectuant les étapes suivantes :

- silanisation des substrats au moyen de glycidyloxypropyltriméthoxysilane ; et
- greffage du bras espaceur constitué par du tétraéthylène glycol.

Ces étapes correspondent aux réactions suivantes :

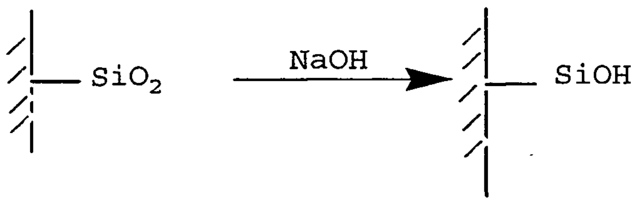


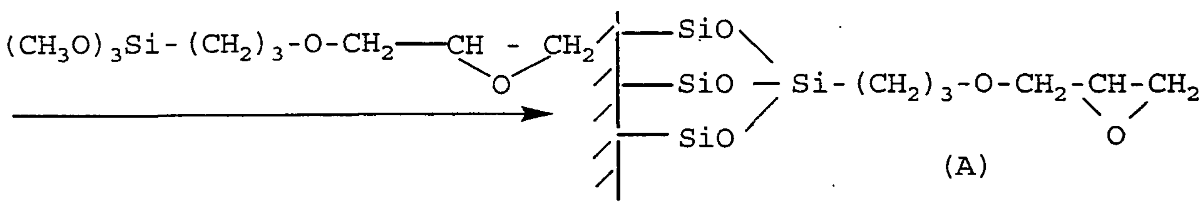

(A)

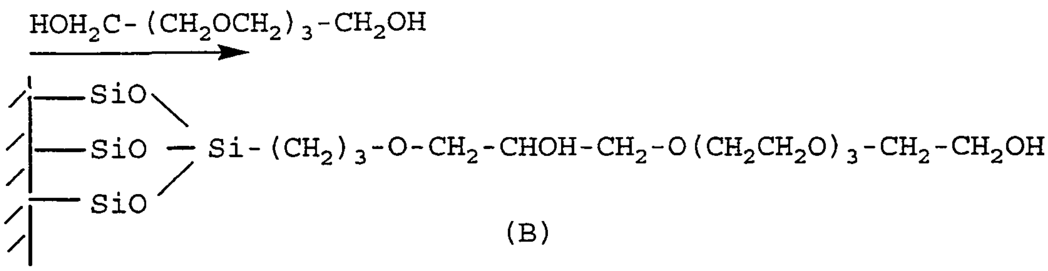


(B)

Ainsi, on soumet tout d'abord les substrats à une silanisation en les immergeant dans une solution comprenant 1 ml de glycidyloxypropyltriméthoxysilane dans 3,5 ml de toluène et 0,3 ml de triéthylamine pendant une nuit à 80°C. On rince ensuite les substrats à l'acétone, on les sèche et on les recuit à 110°C pendant 3 heures.

On obtient ainsi les substrats silanisés (A). On greffe ensuite sur les groupements époxy un bras espaceur afin d'éloigner l'oligonucléotide de la surface et favoriser ainsi les conditions d'hybridation.

Dans ce but, les substrats sont traités en milieu acide pour catalyser la réaction de greffage du bras espaceur constitué par du tétraéthylèneglycol (TEG). On obtient ainsi le substrat traité (B).

Après greffage de ce bras espaceur, on introduit les substrats dans un synthétiseur automatique Expedite 8909 pour réaliser une séquence programmée d'oligonucléotides en utilisant la chimie phosphoramidite. On synthétise ainsi à l'extrémité du bras espaceur des sondes oligonucléotidiques de 20 mères comportant la succession de bases suivante :

3', TTTTT ATC TCA CCC AAA TAG 5'

On utilise ensuite ces différents supports d'analyse pour la détermination de cibles de séquence complémentaire aux sondes synthétisées sur le substrat, ces cibles comportant en position 5' un marqueur fluorescent constitué soit par Cy3, soit par Cy5. On détermine alors l'intensité de fluorescence pour chacun des substrats. Les résultats obtenus sont illustrés sur la figure 3 qui représente l'intensité de fluorescence en fonction de l'épaisseur de la couche de $SiO_2$ (en nm) pour Cy3 et pour Cy5.

Sur cette figure, on voit que pour une épaisseur d'oxyde de 100 nm, on obtient une maximisation des signaux émis par Cy3 et Cy5. En revanche, pour une couche de 190 nm, on obtient une minimisation des signaux émis par Cy3 et Cy5.

Pour une épaisseur de couche de 475 nm, on observe une maximisation du signal émis par Cy3 et une minimisation du signal émis par Cy5.

Pour une épaisseur de couche de 545 nm, on obtient une maximisation de Cy5 et une minimisation de Cy3.

Les résultats expérimentaux obtenus sont donc bien en accord avec les simulations effectuées par calcul sur la figure 2.

### Exemple 2

Dans cet exemple, on utilise un empilement multicouche afin d'équilibrer les rayonnements de fluorescence des fluorophores CY3 et CY5. L'empilement est réalisé en déposant sur un verre la séquence suivante de couches minces à indice de réfraction haut (n= 1,836) et à indice de réfraction bas (n = 1,45):

- une couche de 80nm d'épaisseur en $HfO_2$ (indice de réfraction haut, n = 1,836),
- une couche de 102 nm d'épaisseur en $SiO_2$ (indice de réfraction bas, n = 1,45),
- une couche de 80 nm d'épaisseur en $HfO_2$ (n = 1,836),
- une couche de 102 nm d'épaisseur en $SiO_2$ (n = 1,45),
- une couche de 80 nm d'épaisseur en $HfO_2$ (n = 1,836), et
- une couche de 108 nm d'épaisseur en $SiO_2$ (n = 1,45).

Les réponses théorique et mesurée de cet empilement sont données sur la figure 4 qui illustre l'évolution des propriétés optiques en réflexion (en %) en fonction de la longueur d'onde $\lambda$ (en nm). La courbe 1 se réfère aux valeurs théoriques et les courbes 2 et 3 se réfèrent à trois essais expérimentaux , la courbe 2 illustrant deux essais dont les résultats sont trop proches pour matérialiser les deux courbes.

Au vu de cette figure, on constate que les résultats expérimentaux sont bien en accord avec les simulations effectuées par calcul.

Cet empilement peut être utilisé pour la détection de la mutation d'un gène impliqué dans la synthèse de l'émerine, protéine impliquée dans le déclenchement de la myopathie de Eymery-Dreyfus. Cet empilement est optimisé pour les fluorophores CY3 et CY5.

CY3 : longueur d'onde d'excitation de 550 nm et longueur d'onde d'émission d'environ 580 nm.

CY5 : longueur d'onde d'excitation de 650 nm et longueur d'onde d'émission de 680 nm.

Dans les conditions de lecture du scanner « General Scanning » (ouverture numérique de 0,75), on a des fluorescences théoriques de 0,27 (unité arbitraire) pour les deux fluorophoresCY3 et CY5. Ceci permet d'équilibrer les émissions des deux fluorophores pour le cas particulier de la lecture par le scanner « General Scanning ».

### Références citées

[1] Médecine/Sciences, vol. 13, n°11, 1997, pp. 1317-1324

[2] US-A-5,578,832
[3] US-A-5,646,411
[4] Edward H. Hellen et Daniel Axelrod, J. Opt. Soc. Am. B, vol. 4, n°3, March 1987, pp. 337 à 350
[5] WO 94/22889.

## Revendications

**1.** Procédé de détermination d'un analyte, lequel procédé comprend :

a) la mise en oeuvre d'une réaction spécifique de liaison entre cet analyte et un ligand qui lui est spécifique, l'analyte étant marqué par au moins deux marqueurs fluorescents et le ligand étant fixé sur un support d'analyse, et
b) la détermination de ladite réaction de liaison par application au support d'un champ excitateur pour chacun des marqueurs fluorescents, et détection des signaux fluorescents émis par ces marqueurs ;

et est **caractérisé en ce que** le support d'analyse comprend :

- un substrat,
- une ou plusieurs couches de matériau(x) transparent(s) aux longueurs d'onde d'excitation et d'émission des marqueurs fluorescents, cette ou ces couches revêtant le substrat et formant un ensemble qui atténue ou augmente le champ excitateur de l'un au moins des marqueurs fluorescents de sorte à équilibrer les intensités des signaux fluorescents émis par ces marqueurs ; et
- une pluralité de ligands spécifiques de l'analyte, ces ligands étant fixés sur la surface dudit ensemble.

**2.** Procédé selon la revendication 1, dans lequel l'analyte est une sonde ADN ou ARN et le ligand est la sonde complémentaire ADN ou ARN.

**3.** Procédé selon la revendication 1 ou la revendication 2, dans lequel la ou les couches de matériau(x) revêtant le substrat sont en oxyde réfractaire, en fluorure métallique ou en oxynitrure de silicium.

**4.** Procédé selon la revendication 3, dans lequel l'oxyde réfractaire est choisi parmi $TiO_2$, $HfO_2$, $Ta_2O_5$, $SiO_2$ et $ZrO_2$.

**5.** Procédé selon la revendication 3, dans lequel le fluorure métallique est choisi parmi $YF_3$ et $MgF_2$.

**6.** Procédé selon l'une quelconque des revendications 1 à 5, dans lequel le substrat est en un matériau semi-conducteur, en verre ou en une matière plastique.

**7.** Procédé selon la revendication 1 ou la revendication 2, dans lequel l'analyte est marqué par Cy3 dont la longueur d'onde d'excitation est d'environ 550 nm et la longueur d'onde d'émission est d'environ 580 nm, et par Cy5 dont la longueur d'onde d'excitation est d'environ 650 nm et la longueur d'onde d'émission est d'environ 680 nm.

**8.** Procédé selon la revendication 7, dans lequel ledit ensemble est constitué d'une couche de $SiO_2$ ayant une épaisseur comprise entre environ 100 et 1000 nm.

**9.** Procédé selon la revendication 8, dans lequel le substrat est en silicium.

## Claims

**1.** Method for determining an analyte, which method comprises:

a) carrying out a specific reaction of binding of this analyte with a ligand which is specific for it, the analyte being labelled with at least two fluorescent labels and the ligand being attached onto an analysis support; and
b) determining the binding reaction by applying to the support an excitatory field for each of the fluorescent labels and by detecting the fluorescent signals emitted by these labels;

and is **characterized in that** said analysis support comprises:

- a substrate;
- one or more layers of material(s) transparent to the excitation and emission wavelengths of the fluorescent labels, this or these layers coating the substrate and forming an assembly which lessens or increases the excitatory field for at least one of the fluorescent labels so as to balance the intensities of the fluorescent signals emitted by these labels; and
- a plurality of ligands specific for the analyte, these ligands being attached to the surface of said assembly.

**2.** Method according to claim 1, in which the analyte is a DNA or RNA probe and the ligand is the complementary DNA or RNA probe.

**3.** Method according to claim 1 or claim 2, in which the layer or the layers of material(s) coating the substrate are made of refractory oxide, metal fluoride or silicon oxynitride.

**4.** Method according to claim 3, in which the refractory oxide is chosen from $TiO_2$, $HfO_2$, $Ta_2O_5$, $SiO_2$ and $ZrO_2$.

**5.** Method according to claim 3, in which the metal fluoride is chosen from $YF_3$ and $MgF_2$.

**6.** Method according to any one of claims 1 to 5, in which the substrate is made of a semi-conductor material, of glass or of a plastic material.

**7.** Method according to claim 1 or claim 2, in which the analyte is labelled with Cy3, the excitation wavelength of which is approximately 550 nm and the emission wavelength of which is approximately 580 nm, and with Cy5, the excitation wavelength of which is approximately 650 nm and the emission wavelength of which is approximately 680 nm.

**8.** Method according to claim 7, in which said assembly is composed of a layer of $SiO_2$ having a thickness comprised between about 100 and 1 000 nm.

**9.** Method according to claim 8, in which the substrate is made of silicon.

**Patentansprüche**

**1.** Verfahren zur Bestimmung eines Analyten, wobei das Verfahren

a) das Ausführen einer spezifischen Bindungsreaktion zwischen diesem Analyten und einem für diesen Analyten spezifischen Liganden, wobei der Analyt durch wenigstens zwei Fluoreszenzmarker markiert ist und der Ligand auf einem Analysenträger fixiert ist, und
b) die Bestimmung der Bindungsreaktion durch Anlegen eines Anregungsfelds für jeden Fluoreszenzmarker an den Träger und Nachweis der durch diese Marker emittierten Fluoreszenzsignale umfasst,

und **dadurch gekennzeichnet ist, dass** der Analysenträger

- ein Substrat,
- eine oder mehrere Schichten aus transparentem (transparenten) Material(ien) mit den Anregungs- und Emissionswellenlängen der Fluoreszenzmarker, wobei diese Schicht(en) das Substrat bedecken und eine Anordnung bilden, die das Anregungsfeld wenigstens eines der Fluoreszenzmarker vermindert oder erhöht, um die Intensitäten der durch diese Marker emittierten Fluoreszenzsignale anzugleichen, und
- eine Mehrzahl für den Analyten spezifischer Liganden umfasst, wobei diese Liganden auf der Oberfläche der Anordnung fixiert sind.

**2.** Verfahren gemäß Anspruch 1, wobei der Analyt eine DNA- oder RNA-Sonde ist und der Ligand die komplementäre DNA- oder RNA-Sonde ist.

**3.** Verfahren gemäß Anspruch 1 oder Anspruch 2, wobei die Schicht oder Schichten aus das Substrat bedeckenden Material(ien) aus einem feuerfesten Oxid, Metallfluorid oder Siliziumoxynitrid sind.

**4.** Verfahren gemäß Anspruch 3, wobei das feuerfeste Oxid aus $TiO_2$, $HfO_2$, $Ta_2O_5$, $SiO_2$ und $ZrO_2$ ausgewählt wird.

**5.** Verfahren gemäß Anspruch 3, wobei das Metallfluorid aus $YF_3$ und $MgF_2$ ausgewählt wird.

**6.** Verfahren gemäß einem der Ansprüche 1 bis 5, wobei das Substrat aus einem Halbleitermaterial, Glas oder Kunststoffmaterial ist.

**7.** Verfahren gemäß Anspruch 1 oder Anspruch 2, wobei der Analyt durch Cy3, dessen Anregungswellenlänge etwa 550 nm ist und dessen Emissionswellenlänge etwa 580 nm ist, und durch Cy5, dessen Anregungswellenlänge etwa 650 nm ist und dessen Emissionswellenlänge etwa 680 nm ist, markiert ist.

**8.** Verfahren gemäß Anspruch 7, wobei sich die Anordnung aus einer $SiO_2$-Schicht mit einer Dicke zwischen etwa 100 und 1000 nm zusammensetzt.

**9.** Verfahren gemäß Anspruch 8, wobei das Substrat aus Silizium ist.

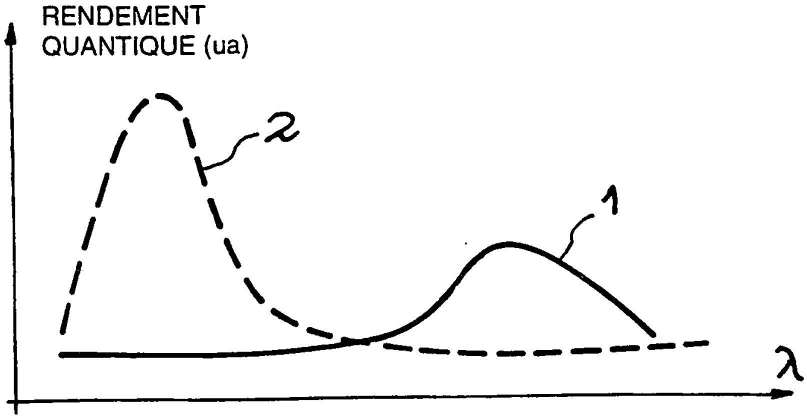


FIG. 1

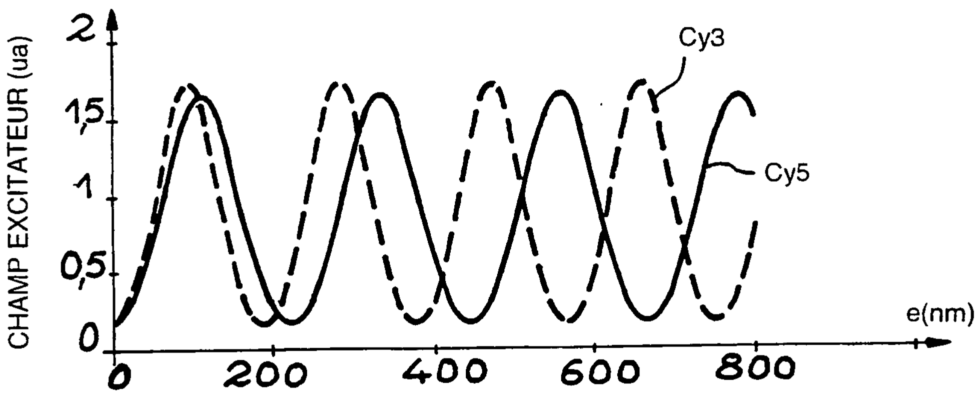


FIG. 2

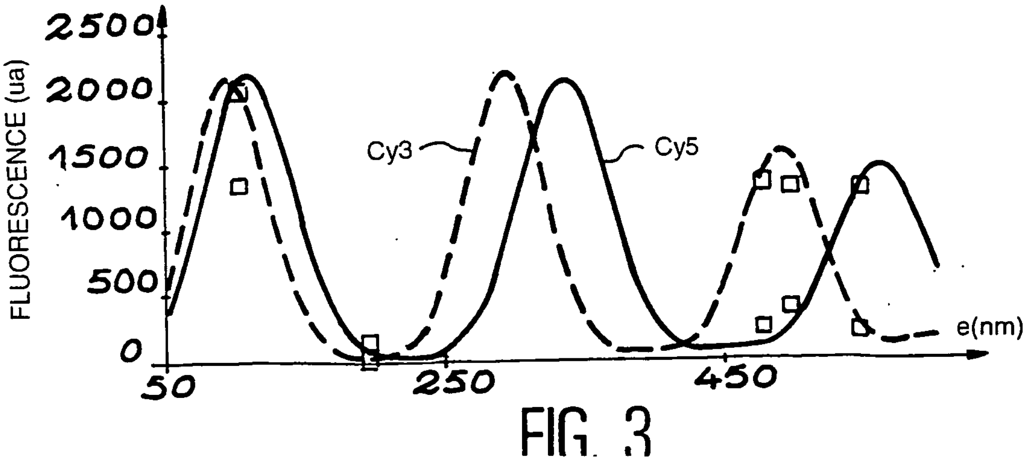


FIG. 3

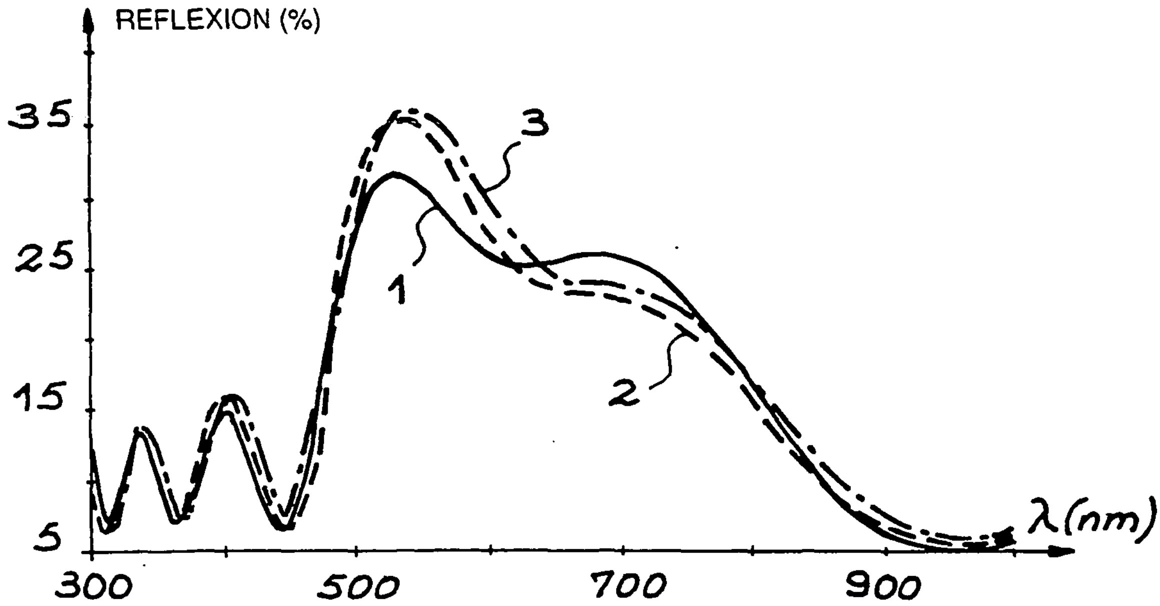


FIG. 4

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- US 5578832 A
- US 5646411 A
- WO 9422889 A


### Littérature non-brevet citée dans la description


- *Médecine/Sciences,* 1997, vol. 13 (11), 1317-1324
- **EDWARD H. HELLEN ; DANIEL AXELROD.** *J. Opt. Soc. Am. B,* Mars 1987, vol. 4 (3), 337-350